# EUROPEAN PATENT APPLICATION

(11) **EP 4 265 278 A1**
(43) Date of publication of application: **25.10.2023**
(21) Application number: 22191827.9
(22) Date of filing: 23.08.2022
(51) Int. Cl.: A61L 2/03, A61L 2/04, H05H 1/20

(54) **DEVICE FOR STERILIZATION AND DISINFECTION OF ITEMS**

(30) Priority: 21.04.2022 EP 22461537
(71) Applicant: Elbiotech Sp. z o.o., 04-083 Warsaw (PL)
(72) Inventor: Pachowicz, Krzysztof, Warsaw (PL); Luniewski, Slawomir, Kajetany (PL); Baraniak, Joanna, Warsaw (PL)
(74) Representative: AOMB Polska Sp. z.o.o.

(57) **Abstract**

The invention relates to the field of sterilization and disinfection of objects, such as documents, money, packagings, or personal hygiene articles. The invention performs an ultra-fast sterilization and disinfection of objects. The device sterilization and disinfecting comprises an inner housing (7), a lid (6), two flat electrodes, clamping means (5), a dielectric layer (3), a chamber (4), a high voltage power supply and a release of electric field impulses, and it is characterized in that the dielectric layer (3) is in a solid form.

## Description

### Field of the invention

The invention relates to the field of sterilization and disinfection of objects, such as documents, money, packagings or personal hygiene articles. More specifically, the invention provides a device and method for ultra-fast sterilization and disinfection of objects.

### Prior art

Document US 5326530 discloses a method for removal of harmful biological organisms from contaminated objects. The contaminated material, flowing from a chamber, is subject to non-uniform electric field of a large intensity. The invention discloses electrodes and the material being sterilized is positioned therebetween, while a pulsed voltage is applied to the electrodes.

Document CN 102512756 discloses a method and equipment for generating high voltage induction current. The grid of shaped impulses releases high voltage impulses of a specific wave shape and wave width, a group of induction coils may cause a change in the magnetic induction flux in a form of an impulse affected by the high voltage impulses, a high voltage induction field in a form of an impulse is induced and generated by the change in the magnetic induction flux, within the space in the vicinity of a group of induction coils, a high intensity induced electromotive force in a pulsed form is generated within the object being subject to treatment and positioned in the induction field. The disclosed solution is intended for sterilization with the use of a magnetic field.

In the prior art devices are known for sterilization of object with the use of electrodes to which a voltage is applied, but none of the devices enables obtaining of high effectiveness of operation while providing very fast operation.

### Subject-matter of the invention

It is the aim of the invention to provide a solution that enables high effectiveness of sterilization and disinfecting of object in a very short time.

According to the invention there is provided a device for sterilization and disinfecting, comprising an inner housing, a lid, two flat electrodes, one being secured on the lid and the other one in the inner housing, a dielectric layer, a chamber positioned between a flat electrode and the dielectric layer, a high voltage power supply, and a release of electric field impulses, which impulses are capable to generate pulsed high voltage on a high voltage electrode, the high voltage power supply being connected to the impulse release, and the impulse release is connected to a high voltage electrode, being characterized in that it comprises clamping means positioned on the inner housing and on the lid, and a dielectric layer in a solid form.

Preferably, the dielectric layer is positioned in the inner housing.

In one of the preferable embodiments, the clamping means is constituted by at least one of a set of magnets and electromagnets or snap fasteners.

In one of the examples at the at least one flat electrode there is a heater.

Preferably, the heater is an etched path on a first surface of a printed circuit board, while preferably on the other surface of the printed circuit board PCB there being a flat electrode.

Preferably, it comprises a Faraday shield that encircles the flat electrodes in a closed position, and preferably the Faraday shield has a form of the inner housing and the lid.

In one of the examples, the inner housing is connected with the lid via hinges or mechanic snap fasteners.

At least one electrode is made of a metal sheet, preferably a copper sheet.

Preferably, at least one electrode is positioned on an elastic material, preferably on an elastic polymer, more preferably on a EPDM gum.

The metal sheet has a thickness of less than 1 mm, preferably 0.1 mm.

The elastic material has a thickness of less than 10 mm, preferably 5 mm.

In one of the preferable embodiments, relative electric permittivity of the dielectric layer is close to the relative electric permittivity of the object subjected to sterilization and disinfection, and preferably it amounts to about εᵣ = 3.

Preferably, the dielectric layer is silicon.

One of the examples comprises a set of proximity sensors, preferably being magnetic proximity sensors.

In one of the preferable embodiments, in the closed position, the dielectric layer adjoins directly the flat electrode.

Preferably, the power supply is configured to generate impulses the rise time of which is 10 to 300 ns, and duration time is 1 to 50 µs.

### Advantageous effects of the invention

The proposed invention enables obtaining maximum operative effectiveness during sterilization and disinfection within a very short time, and enhancing effectiveness of the process via a possibility of heating to a temperature higher than the room temperature. The invention preferably enables fast operation, of an order of several seconds, and simplicity of operation. The invention enables its use, in all cases where there is a necessity for fast disinfection of objects, and restrains migration of pathogens.

### Brief description of the drawings

The invention shall be presented below with reference to the enclosed drawings in which:
- Fig. 1 -: shows an internal scheme of the device,
- Fig. 2 -: shows a device in an isometric view,
- Fig. 3 -: shows a device in a top view, without a lid,
- Fig. 4 -: shows an open device in an isometric view,
- Fig. 5 -: shows an exemplary shape of an impulse.

### Detailed description of a preferable embodiment of the invention

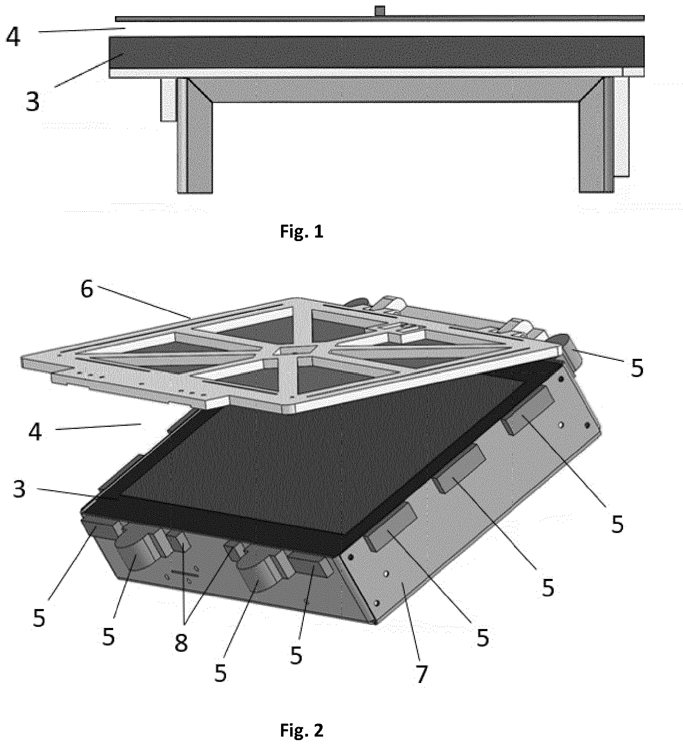

With regard to the enclosed Figs. 1-5, technical aspects of the device and method for sterilization and disinfecting of object will be elucidated.

Figs. 1 and 2 show schematically the inner appearance of a device. The device is constructed of an inner housing 7, a lid 6, two flat electrodes, clamping means 5 positioned in the inner housing 7 and the lid 6, a dielectric layer 3, a chamber 4 which is positioned between the two flat electrodes, a high voltage power supply and an electric field impulse release connected thereto. In this embodiment one of the flat electrodes, the high voltage electrode, is mounted in the inner housing 7, while the other electrode grounded with the heater, in the lid 6. In another embodiment positioning of the flat electrodes may be reversed. The high voltage power supply and the electric field impulse release generate pulsed high voltage on the high voltage electrode. The impulse release is connected to the high voltage electrode.

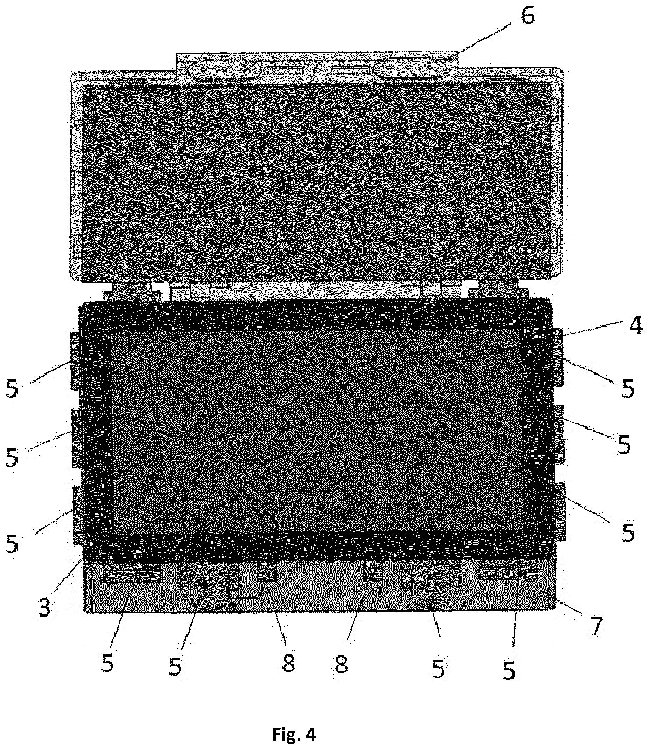

In this embodiment the high voltage electrode is made of elastic materials. Between the flat electrodes there is a dielectric layer 3 in a solid form, which dielectric being selected so that it is elastic and has a low value of electric permittivity. The dielectric layer 3 made of a material in a solid form, is positioned in the inner housing 7. Within the chamber 4 the object to be sterilized and disinfected is positioned. Suitably to the size of the chamber 4 and its operational area, different object subjected to disinfection are positioned therein. The object is positioned in a simple manner within the chamber 4 due to movable positioning of the second flat electrode in the lid 6, such as it is shown in Fig. 4. In this embodiment the dielectric layer 3 is preferably silicon.

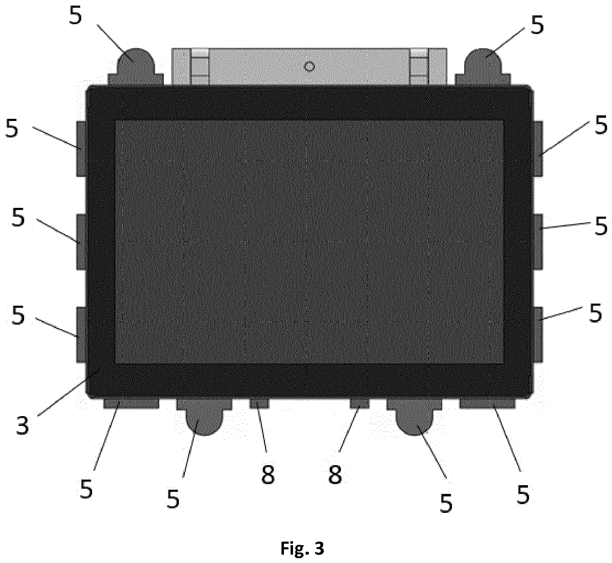

The device is equipped with clamping means 5, such as an arrangement of magnets, electromagnets or snap fasteners, as shown in Fig. 3. Such presented arrangement of clamping means 5, around the operational space of the chamber 4 enables closing of the lid 6 against the inner housing 7 with a high and sufficient force necessary for accurate sterilization and disinfection of the object positioned within the chamber 4. The clamping means 5 enable close abutment of the flat electrode positioned in the lid 6 against the sterilized material, without spaces in the chamber 4 that would be filled with air. Applying of high voltage to the flat electrode, which is the high voltage electrode, causes generation of an electric field between the two flat electrodes, the high voltage electrode and the grounded electrode. The use of clamping means 5 enables uniform distribution of the electric field in the chamber 4 with the object being disinfected positioned therein, and at the same time enlarges the value of the electric field. This construction preferably enables faster and more uniform heating of the entire operational space in the chamber 4 and lowers the probability of propagation of electric fields disturbances.

The second flat electrode, in this embodiment grounded with the heater, is made in this embodiment on one surface of a printed circuit board PCB, being in this embodiment a FR4 laminate covered at two sides with copper, and a person skilled in the art will be aware that the heater may be also made with the use of some other technology. The flat electrode, in the closed position of the device, where the lid 6 clamps the object being sterilized in the chamber 4 via the clamping means 5, contacts with one surface the object being sterilized. In this embodiment, in the closed position, the dielectric layer abuts directly against the flat electrode. In this embodiment this adjoining surface is covered with solid copper and is connected to the ground. The other side of the flat electrode, the one which does not contact the object being sterilized, is covered with copper that is etched so that a heating circuit is formed on the area of the flat electrode, to cover the entire operational area of the chamber 4. The size of the area is selected so that the heating circuit has a suitable resistance. Applying current to the etched path causes warming up of the heating circuit and thus heating of the entire flat electrode and the object subject to be sterilized, in the closed position of the device. Control of the temperature of the hearing circuit is performed by means of an NTC thermistor positioned in the middle of the operational area of the chamber 4, at one side of the heating circuit, and a person skilled in the art will be aware of other methods for adjustment of the heater temperature.

The device in this embodiment comprises a Faraday shield that encloses the flat electrodes in the closed position of the device. In this example, the Faraday shield is constituted by the inner housing 7 and the lid 6 and it is made of a steel sheet, and also an embodiment of the device is possible in which the Faraday shield will be at the same time constitute the outer housing of the device. In the closed position of the device it does not enable the electromagnetic radiation to escape out of the device. In other embodiments, the Faraday shield constitutes the inner housing 7 and the lid 6 is positioned in the outer housing, which outer housing mainly meets aesthetics requirements and serves as a place where a display and used press buttons are positioned. The inner housing 7 and the lid 6 are mutually connected in this embodiment by means of hinges or mechanic snap fasteners. Also an embodiment of the device is possible where the inner housing 7 and the lid 6 are two separate elements that form together one entirety only in the operational position. A person skilled in the art will be aware that it is important to provide a lid 6 and the inner housing 7 so that the flat electrodes are close to each other, and he will know how a device that enables positioning of the flat electrodes in their target location should be designed.

The sizes of the electrodes and the level of elasticity of the material on which the electrode is positioned, are selected suitably to the object being sterilized. In this embodiment the flat electrode is made of a thin copper sheet, of a thickness of 0.1 mm and it is positioned on a mat of EPDM gum of a thickness of 5 mm. The material of the dielectric layer 3 is selected to that its relative electric permittivity is close to the relative permittivity of the object being sterilized and amounts to about εᵣ = 3.

The device comprises an array of proximity sensors 8, shown in Figs. 2, 3 and 4, that provide safety for users during operation of the device. In this embodiment, the array of the proximity sensors 8 are magnetic proximity sensors that are capable to detect improper closing of the lid of the device ant thus improper adherence of the flat electrode to the material being sterilized and disinfected. The array of proximity sensors 8 is responsible for securing against initiation of generation of impulses, when the chamber 4 is open or a too thick object has been inserted thereto that does not make it possible to close the lid 6 completely against the inner housing 7. The array of proximity sensors 8, in this example, is positioned in the vicinity of the edge of the chamber 4, opposite to the one on which the hinges or mechanic snap fasteners are positioned to connect the inner housing 7 to the lid 6.

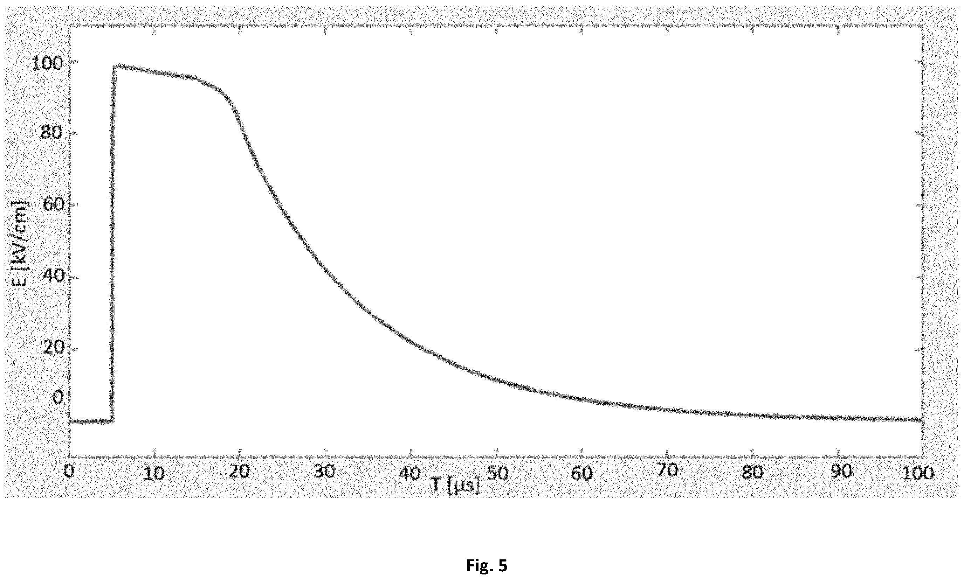

The device enables stepless regulation of the parameters of the electric field impulses that constitute the sterilizing factor. The regulation enables matching the respective parameters with the object being sterilized. It is possible to adjust the length of the impulses within the range of from a few microseconds to several thousands microseconds or milliseconds, as well as their frequency and their number. In this embodiment the constant values are: the peak value of the impulse and the impulse rise time. This is caused by effectiveness of sterilization which should not be affected by the thickness and the structure of the object being sterilized. The peak value of the impulse, generated by the high voltage power supply enables obtaining an electric field of a value of above 10⁵ V/cm, and the impulse rise time is from 10 to 300 ns, and the impulse duration time is 1-50 µs. Regulation is effected by switching off and switching on a high voltage connector that constitutes the release of the electric field impulses. Exemplary impulse shape is shown in Fig. 5.

The device as presented in the text above and in the figures is used for a method for realisation and disinfection of objects. The method comprises the following steps:
a) positioning of an object for sterilization and disinfecting within the chamber (4),
b) placing of the lid (6) on the inner housing (7),
c) applying at least one high voltage impulse to the high voltage electrode that causes an electric field to be generated between the flat electrodes, and the parameters of the magnetic field are regulated by means of a high voltage power supply and impulse release.

## Claims

1. Device for sterilization and disinfection, comprising
an inner housing (7),
a lid (6),
two flat electrodes, one being secured on the lid (6), and the other one in the inner housing (7),
a dielectric layer (3),
a chamber (4), positioned between a flat electrode and the dielectric layer (3),
a high voltage power supply and an electric field impulses release, said impulses being suitable to generate pulsed high voltage and to generate pulsed high voltage on the high voltage electrode,
the high voltage power supply being connected to the impulse release, and the impulse release is connected to the high voltage electrode,
**characterized in that** it comprises clamping means (5) being positioned within the inner housing (7) and on the lid (6), and a dielectric layer (3) in a solid form.

2. Device according to claim 1 **characterized in that** the dielectric layer (3) is within the inner housing (7).

3. Device according to claims 1 or 2 **characterized in that** the clamping means (5) are at least one of a set of magnets and electromagnets or snap fasteners.

4. Device according to any of claims 1-3 **characterized in that** at the at least one flat electrode there is a heater.

5. Device according to claim 4 **characterized in that** the heater is an etched path in the first surface of a printed circuit board PCB, preferably on the other surface of the printed circuit board PCB the flat electrode is positioned.

6. Device according to any of claims 1-5 **characterized in that** it comprises a Faraday shield that encircles the flat electrodes in the closed position, and preferably the Faraday shield has a form of the inner housing (7) and the lid (6).

7. Device according to any of claims 1-6 **characterized in that** the inner housing (7) is connected to the lid (6) be means of hinges or mechanic snap fasteners.

8. Device according to any of claims 1-7, **characterized in that** at least one electrode is made of a metal sheet, preferably of a copper sheet.

9. Device according to of claim 8, **characterized in that** at least one electrode is positioned on an elastic material, preferably on an elastic polymer, more preferably on a EPDM gum.

10. Device according to any of claims 8 or 9, **characterized in that** the metal sheet has a thickness smaller than 1 mm, preferably 0.1 mm.

11. Device according to any of claims 8, 9 or 10, **characterized in that** the elastic material has a thickness smaller than 10 mm, preferably 5 mm.

12. Device according to any of claims 1-11, **characterized in that** the relative electric permittivity of the dielectric layer (3) is close to the relative electric permittivity of the object being sterilized and disinfected, and preferably amounts to about εᵣ = 3, and preferably the dielectric layer (3) is silicone.

13. Device according to any of claims 1-12 **characterized in that** it comprises a set of proximity sensors (6), preferably being magnetic proximity sensors.

14. Device according to any of claims 1-13 **characterized in that** in the closed position the dielectric layer abuts directly the flat electrode.

15. Device according to claim 1 **characterized in that** the high voltage power supply is configured to generate impulses the rise time of which is 10 to 300 ns, and duration time is 1 to 50 µs.
